# EUROPEAN PATENT APPLICATION

(11) **EP 1 820 493 A2**
(43) Date of publication of application: **22.08.2007**
(21) Application number: 07002816.2
(22) Date of filing: 09.02.2007
(51) Int. Cl.: A61K 9/00

(54) **Aerosols for sinunasal drug delivery**

(30) Priority: 10.02.2006 EP 06002735
(71) Applicant: PARI Pharma GmbH, 82319 Starnberg (DE)
(72) Inventor: Schuschnig, Uwe., 81371 München (DE); Keller, Manfred, 81379 M[nchen (DE)
(74) Representative: Beckmann, Claus

(57) **Abstract**

A pharmaceutical aerosol is disclosed which is suitable for delivering an active compound to the mucosa of the nasal cavity or of the paranasal sinuses. The aerosol is characterised in that its pressure is not constant, but pulsates at a frequency of about 10 to 90 Hz. The mass median diameter of the dispersed phase is from about 2 to 6 µm, and the volume of about 5 mL or less of the dispersed phase comprises a unit dose of an active compound. The aerosol is, inter alia, suitable for the prevention, management, or treatment of a disease, symptom, or condition affecting the nose or the paranasal sinuses, such as acute and chronic sinusitis.

## Description

### FIELD OF THE INVENTION

The present invention is related to pharmaceutical aerosols and to liquid compositions which are suitable for aerosolisation. In further aspects, the invention relates to therapeutic uses of aerosols and to methods of producing them and of administering them to patients. The aerosols are suitable for delivering drug substances to selected regions within the respiratory tract, including the nasal cavity and the paranasal sinuses.

### BACKGROUND OF THE INVENTION

Diseases and conditions affecting either paranasal sinuses or both the nasal cavity and the paranasal sinuses, in particular acute and chronic forms of rhinosinusitis, are increasing in incidence and prevalence in many countries and regions of the world, including Europe and the United States. These conditions may be associated with significant symptoms and have a negative impact on quality of life and daily functioning.

On the other hand, the effective treatment of the nasal and paranasal mucosa remains challenging. While the mucosa of the nasal cavity is a feasible target for locally administered drugs formulated as nasal sprays, the sinuses are not easily accessed by liquid formulations. In the case of relatively coarse aerosols, such as conventional nasal sprays, the deposition on the sinus mucosa is negligible, and even finer aerosols, such as those generated by nebulisers, exhibit a very low degree of sinus deposition.

The primary reason for the lack of access of an inhaled aerosol to the sinuses is anatomical: in contrast to the nasal cavity, the sinuses are not actively ventilated. They are connected to the nasal passage via small orifices called ostia, whose diameter is typically in the region of only about 0.5 to 2 mm. When air is inhaled through the nose and passes through the nasal passage into the trachea, there is only very little convective flow into the ostia.

To address the need for devices and methods which are more effective in delivering an aerosol to the paranasal sinuses, it was suggested in WO 2005/023335 that certain particle size and velocity characteristics must be achieved in order that a majority of an aerosolised drug formulation reaches the deep nasal cavities and the sinuses.

Furthermore, WO 2004/020029 discloses an aerosol generator comprising a nebuliser and a compressor which delivers a vibrating stream of air to the nebuliser. The document further describes that the aerosol emitted from the nebuliser should be inhaled through one nostril via an appropriate nosepiece, and that the other nostril should be closed by an appropriate device.

However, it has been found by the inventors that these teachings of the prior art do not actually ensure the deposition of a large fraction on the sinunasal mucosa, depending on the actual configuration of the devices and the aerosol characteristics. Thus, there remains a need for further improvements with regard to the aerosol generators and the aerosol formulations in order to allow an improved therapy of sinunasal diseases and conditions.

It is therefore an object of the present invention to provide improved pharmaceutical aerosols which are useful for delivering active compounds to the mucosa of the paranasal sinuses or of both the nasal cavity and the paranasal sinuses. Furthermore, it is an object of the invention to provide methods for producing such aerosols. Further objects will become clear on the basis of the following desciption and the patent claims.

### SUMMARY OF THE INVENTION

In a first principal aspect, the invention provides an aerosol for delivering an active compound to the mucosa of the nose or of a paranasal sinus. The aerosol comprises a dispersed liquid phase and a continuous gas phase. The volume of the dispersed liquid phase which contains a unit dose of the active compound is less than about 5 mL. The mass median diameter of the dispersed liquid phase is from about 2.0 to about 6.0 µm. Furthermore, the pressure of the aerosol pulsates with a frequency in the range from about 10 to about 90 Hz.

In a second principal aspect, the invention provides a method for producing a pharmaceutical aerosol for the delivery of an active compound to the mucosa of the nose or of a paranasal sinus. Again, the aerosol comprises a dispersed liquid phase and a continuous gas phase. The method involves the steps of (a) providing an aerosol generator capable of emitting an aerosol whose pressure pulsates with a frequency in the range from about 10 to about 90 Hz, wherein the aerosol generator is adapted to maintain an amplitude of pressure pulsation of the emitted aerosol of at least about 10 mbar, (b) providing a liquid composition comprising said active compound, wherein a unit dose of the active compound is comprised in a volume of less than about 5 mL of said liquid composition, and (c) aerosolising said liquid composition.

The method is preferably practised with an aerosol generator which is capable not only of delivering an aerosol which pulsates at the specified frequency, but which is also capable to maintain an amplitude of pressure pulsation of at least about 10 mbar. The aerosol generator comprises a nebuliser which may, for example, be an electronic vibrating membrane nebuliser or a jet nebuliser connected to a suitable air compressor.

The active compound may be selected from various therapeutic categories, such as from anti-inflammatory compounds, glucocorticoids, anti-infective agents, antibiotics, antifungals, antivirals, mucolytics, antiseptics, wound healing agents, local anaesthetics, peptides, and proteins.

The method of the invention, and the aerosols thus produced, achieve a high deposition of drug in the nasal cavities and/or paranasal sinuses. Thus, they are preferably used for the prevention, management, or treatment of a disease, symptom, or condition selected from acute and chronic sinusitis, such as allergic sinusitis, seasonal sinusitis, bacterial sinusitis, fungal sinusitis, viral sinusitis, frontal sinusitis, maxillary sinusitis, sphenoid sinusitis, ethmoid sinusitis, vacuum sinusitis; acute and chronic rhinitis, such as allergic rhinitis, seasonal rhinitis, bacterial rhinitis, fungal rhinitis, viral rhinitis, atrophic rhinitis, vasomotor rhinitis; any combination of rhinitis and sinusitis (i.e. rhinosinusitis); nasal polyps, nasal furuncles, epistaxis, wounds of the nasal or sinunasal mucosa, such as after injury or surgery; and dry nose syndrome.

In further embodiments, the active compound is poorly water-soluble, and formulated with the use of particular formulation techniques in order that the volume of liquid which contains a unit dose is not more than about 5 mL, and that the duration of aerosol generation and administration is still convenient to the patients. Such formulation techniques include drug nanoparticles, colloidal carrier systems, or the use of solubility-enhancing agents. Preferably, the duration of administration is not more than about 30 minutes, or not more than about 20 minutes according to another embodiment.

Further embodiments of the invention will become obvious on the basis of the following detailed description, the examples and the patent claims.

### DETAILED DESCRIPTION OF THE INVENTION

In a first aspect, the invention provides a pharmaceutical aerosol for the delivery of an active compound to the mucosa of the nose or of a paranasal sinus comprising a dispersed liquid phase and a continuous gas phase. The volume of the dispersed liquid phase comprising a unit dose of the active compound is less than about 5 mL. The mass median diameter of the dispersed liquid phase is from about 2.0 to about 6.0 µm, as measured by laser diffraction. Furthermore, the pressure of the aerosol pulsates with a frequency in the range from about 10 to about 90 Hz.

In a second principal aspect, the invention provides a method for producing a pharmaceutical aerosol for the delivery of an active compound to the mucosa of the nose or of a paranasal sinus, said aerosol comprising a dispersed liquid phase and a continuous gas phase. The method comprises the steps of (a) providing an aerosol generator capable of emitting an aerosol whose pressure pulsates with a frequency in the range from about 10 to about 90 Hz, wherein the aerosol generator is adapted to maintain an amplitude of pressure pulsation of the emitted aerosol of at least about 10 mbar, (b) providing a liquid composition comprising said active compound, wherein a unit dose of the active compound is comprised in a volume of less than about 5 mL of said liquid composition, and (c) aerosolising said liquid composition.

As used herein, an aerosol is a dispersion of a solid or liquid phase in a gas phase. The dispersed phase, also termed the discontinuous phase, is comprised of multiple solid or liquid particles. Typically, the particle size of the dispersed phase is less than about 100 µm, and considerably less than that. Both basic physical types of aerosols, i.e. solid and liquid dispersions in a gas phase, may be used as pharmaceutical aerosols. Examples of aerosols representing solid particles in a gas phase or those emitted by dry powder inhalers (DPI's). In contrast, pressurised metered-dose inhalers and nebulisers deliver aerosols whose dispersed phase is liquid.

According to the present invention, the aerosol comprises a dispersed liquid phase and a continuous gas phase. Such aerosols are sometimes referred to as "liquid aerosols" or, probably more appropriately, aerosolised liquids. It should be noted that the requirement of a dispersed liquid phase does not exclude the presence of a solid phase. In particular, the dispersed liquid phase may itself represent a dispersion, such as a suspension of solid particles in a liquid.

The continuous gas phase may be selected from any gas or mixture of gases which is pharmaceutically acceptable. For example, the gas phase may simply be air or compressed air, which is most common in inhalation therapy using nebulisers as aerosol generators. Alternatively, other gases and gas mixtures, such as air enriched with oxygen, or mixtures of nitrogen and oxygen may be used. Most preferred is the use of air as continuous gas phase.

An active compound is a natural, biotechnology-derived or synthetic compound or mixture of compounds useful for the diagnosis, prevention, management, or treatment of a disease, condition, or symptom of an animal, in particular a human. Other terms which may be used as synonyms of active compound include, for example, active ingredient, active pharmaceutical ingredient, drug substance, drug, and the like.

The aerosol of the invention is for the delivery of an active compound to the mucosa of the nose or of a paranasal sinus. The nose is, anatomically, a protuberance in vertebrates that houses the nostrils, or nares, which admit and expel air for respiration. In humans, as in most other mammals, it also houses the nose hairs, which catch airborne particulate contaminants and prevent them from reaching the lungs. Within and behind the nose is the olfactory mucosa and the paranasal sinuses. Behind the nasal cavity, air next passes through the pharynx, shared with the digestive system, and then into the rest of the respiratory system.

The paranasal sinuses consist of four pairs of air-filled cavities or spaces within the bones of the skull and face. They are divided into subgroups which are named according to the bones they lie under: (1) the maxillary sinuses, also called the antra, which are located under the eyes, in the upper jawbone; (2) the frontal sinuses, which lie above the eyes, in the bone of the forehead; (3) the ethmoid sinuses, positioned between the nose and the eyes, backwards into the skull; and (4) the sphenoid sinuses, which are more or less in the centre of the skull base. While the primary function of the sinuses is not entirely clear, it appears that they decrease the relative weight of the front of the skull, warm and humidify the inhaled air before it reaches the lungs, increase the resonance of the voice, and perhaps provide a buffer against blows to the face.

The nasal cavity and the paranasal sinuses are lined with mucosa. Mucosae, or mucous membranes, are mucus-covered epithelial linings. The mucosae of the nasal cavity and the paranasal sinuses are often affected by conditions such as allergies and infections, and the aerosol of the invention provides improved means to deliver therapeutically useful active agents to these membranes.

According to the invention, the volume of the dispersed liquid phase of the aerosol which comprises a unit dose of the active compound is less than about 5 mL. A unit dose is understood as the quantity of an active compound which is suitable and effective when given at one event of administration. Optionally, a unit dose, or single dose, is administered repeatedly, according to a certain regimen, such as once daily, twice daily, or three times daily, for an extended period of time, such as several days, weeks, or even longer.

In one of the embodiments, the volume of the dispersed liquid phase which comprises a unit dose, or a single dose, of the active compound is in the range from about 0.5 mL to about 4.5 mL. It has been found that, in contrast to pulmonary aerosol therapy in which the administration of 5 mL of liquid phase are not uncommon, a better chance for the delivery of a higher portion of the drug contained in the aerosol to mucosa of the nasal cavity and the paranasal sinuses is achieved by selecting relatively low volumes. Without wishing to be bound by theory, this effect is believed to be related to a limited capacity of particularly the paranasal mucosae to hold the deposited aerosol. In other words, the higher the volume of the liquid phase administered to the target mucosae, the higher the likelihood that a substantial fraction of the aerosol will be drained or discharged before it can become effective. Thus it is possible that higher volumes change the distribution pattern of the deposited aerosol: if a small volume, such as 2 mL of liquid phase, is deposited onto the sinunasal mucosae according to a certain useful or desirable pattern, such pattern may be altered substantially if the volume is increased to 5 mL or more.

In further preferred embodiments, the volume of the dispersed phase is about 4 mL or less, 3.5 mL or less, 3 mL or less, 2.5 mL or less, or in the range from about 0.5 to about 3 mL, or from about 1 to about 2.5 mL. It should be noted that, in calculating the volume of the liquid phase which is aerosolised, many of the currently available aerosol generators have a dead volume of up to about 1 mL, some of them even more than 1 mL, so that a larger volume of liquid must be filled into the fluid feed of the device to obtain a certain volume of aerosolised liquid.

According to the invention, the mass median diameter (MMD) of the dispersed liquid phase is from about 2.0 to about 6 µm, as measured by laser diffraction. Various appropriate analytical apparatuses to determine the mass median diameter are known and commercially available, such as the Malvern MasterSizer X or Malvern SprayTec. The geometric distribution of the aerosolised liquid particles or droplets may be determined simultaneously with the mass median diameter. In some embodiments, also the geometrical standard deviation which characterises the broadness of the size distribution of the aerosol particles is of significance, as will be shown in more detail further below.

While the mass median diameter should be rather small, such as less than about 3 µm, or even less than about 2 µm, if the deep lung is the targeted site of aerosol delivery, such as in those cases where systemic absorption of an active compound through the lungs is desired, it has been found that the most useful diameter for depositing the aerosol in the nasal cavity and in the paranasal sinuses may be somewhat larger. For example, a MMD in the region of 3 to 3.5 µm does not seem very desirable for pulmonary delivery, but may be suitable for sinus delivery. Furthermore, it is suggested that the MMD which will lead to the relatively largest aerosol deposition may also depend on individual factors, in particular on the geometry of the paranasal sinuses including the ostia through which the aerosol reaches the sinuses. For example, the volume of the sinuses and the diameter of the ostia differ substantially between individuals. A larger diameter of the ostia is believed to favour the entrance of larger aerosol droplets into the sinuses, even though the diameters of the ostia and of the droplets are of completely different magnitudes. If the individual sinunasal anatomy, or a parameter derived therefrom, of a person to be treated with an aerosol is at least partially known, it may even be possible to select a particular MMD for optimised sinunasal or sinus delivery. In some embodiments, the aerosol of the invention may have a mass median diameter of about 2.5 to 4.5 µm, in others from about 3 to about 4 µm, or from about 2.8 to about 3.5 µm, respectively. In further embodiments, the MMD is approximately (± 0.2 µm) 2.8 µm, 3.0 µm, 3.2 µm, 3.4 µm, 3.6 µm, 3.8 µm, or 4.0 µm.

One of the key features of the aerosol is that it pulsates, or vibrates, with a selected frequency. As used herein, the pulsation of an aerosol is understood as a periodic change of pressure. Preferably, the pulsation is regular, i.e. the time interval between pressure peaks is approximately constant. The amplitude of pressure pulsation may also be relatively constant, at least with regard to the generation and emission of the pulsating aerosol from the aerosol generator.

According to the invention, the pressure of the aerosol pulsates with a frequency in the range from about 10 Hz to about 90 Hz. According to some of the presently preferred embodiments, the pressure may also pulsate at a frequency in the range from about 10 to about 60 Hz, or from 10 to about 55 Hz, or from about 30 to about 60 Hz. In a further embodiment, the aerosol vibrates at a frequency of about 30 to about 55 Hz, such as from about 40 to about 50 Hz, for example about 44 Hz.

It has been found that a vibrating aerosol enters the paranasal sinuses after nasal inhalation to a much larger extent than a conventional aerosol having a substantially constant pressure, provided that the appropriate particles sizes are selected as outlined above. Larger particle sizes will lead to little sinus deposition, but to a large deposition on the nasal mucosa, whereas very small particle sizes allow the aerosol droplets to enter the sinuses following the pressure gradient of a pressure pulse, but also their exit from the sinuses without them being deposited therein.

The principle of generating and applying a pulsating or vibrating aerosol for enhanced sinus deposition has recently been found and described, for example, in EP-A 0 507 707 and WO 2004/020029, whose entire disclosures are incorporated herein by reference.

The paranasal sinuses are, under normal circumstances, poorly ventilated during breathing. Most of the air exchange of the sinuses occurs through the diffusion of air through the ostia, whereas little or no convective flow is observed. If an aerosol, such as a therapeutic aerosol generated by a conventional nebuliser, is inhaled through the nose, the aerosol will flow through the nasal cavity to the lower respiratory tract, if it comprises particles with an appropriately small diameter. Since there is virtually no active flow into the paranasal sinuses, very little or almost none of the aerosol is deposited therein.

In contrast, an aerosol flow which is superimposed with pressure fluctuations, or pressure pulses, creates periodic transient pressure gradients extending from the actively ventilated nasal cavity through the ostia to the sinuses, which gradients cause a short period of convective flow of air and aerosol into the sinuses until the pressure therein has become equal to the air pressure in the nasal cavity. A portion of the aerosol droplets which thus enter the paranasal sinuses are deposited therein onto the mucosa. The extent to which the aerosol is deposited depends e.g. on the droplet size. Droplets that are smaller than the preferred particle size are relatively likely to be expelled from the sinuses during the subsequent pulsation phase in which the aerosol pressure, and thus the pressure in the nasal cavity, is lower than the pressure within the sinuses, and during which a convective flow of air from the sinuses to the nasal cavity occurs.

In order that an effective flow of air and aerosol into the paranasal sinuses is induced, it is not only important to generate the pulsating aerosol with an appropriate device which is capable of emitting such aerosol, such as the PARI SINUS (including PARI LC Star, PARI LL and PARI Sprint) nebuliser families whose compressors are adapted to generate pressure pulses of appropriate frequency and altitude, as will be further discussed below.

In order to transmit effectively aerosol pulsation to the nasal cavity it is essential to observe an appropriate inhalation technique. In particular, it is preferred that the aerosol is introduced through one nostril via a nosepiece which seals the nostril from the external air. Secondly, it is preferred to add a resistance means to the exit nostril by means of an appropriate nosepiece or nose plug, in order to maintain a higher pressure amplitude of the pulsating aerosol in the nasal cavities, as is described in WO 2004/020029. Moreover, it is recommended that the person receiving the aerosol closes the soft palate in order to prevent the aerosol from entering the oral cavity.

Preferably, the aerosol generator is adapted to produce and emit a pulsating aerosol and to maintain an amplitude of pressure pulsation of the emitted aerosol of at least about 5 mbar. It has been found that, depending on the individual sinunasal anatomy of a human person, the pressure amplitude of a pulsating aerosol may be attenuated substantially, such as by large sinus volumes. According to this preferred embodiment, however, the aerosol generator is adapted or selected to maintain a pressure amplitude of at least 5 mbar, measured at aerosolflow in the nasal cavity, irrespective of the individual anatomy of the patient.

As the delivery of aerosol to the sinuses is driven by the pressure gradient between the nasal cavity and the sinuses created by the pressure pulses of the vibrating aerosol, the aerosol fraction deposited in the sinuses may be improved if the amplitude of the pulsation is also further increased. Thus, further embodiments of the invention are characterised in that the aerosol generator is adapted to maintain a pressure pulsation amplitude of at least about 10 mbar, or at least about 15 mbar, or at least about 20 mbar, or at least about 25 mbar. Further examples of useful amplitudes maintained by the device are from about 20 to about 50 mbar, or from about 30 to about 50 mbar, such as about 40 mbar. Even higher amplitudes than 50 mbar might be useful for certain patients and indications in which some degree of discomfort to the patients may be found acceptable, such as serious diseases and affections of the sinus mucosae.

As used herein, an aerosol generator is a device or a combination of devices capable of generating and emitting an aerosol. According to the present invention, the device is capable of aerosolising a liquid material into a dispersed liquid phase. Typically, such device is referred to as a nebuliser. Depending on the type and model of the device, the aerosol generator of the invention may require or include a compressor. In other words, the term aerosol generator is used for the complete apparatus or assembly required to produce and emit an aerosol and to administer the aerosol to an animal, such as to a human patient.
Appropriate aerosol generators for practising the invention potentially include nebulisers from any class of presently known nebulisers, such as jet nebulisers, ultrasonic nebulisers, or piezoelectric or vibrating membrane-type nebulisers. Presently preferred are jet nebulisers that are adapted to emit a pulsating aerosol, and that are combined with an appropriate compressor which is capable of delivering pressurised air whose pressure fluctuates with a frequency of about 10 to about 90 Hz. One of the preferred aerosol generators is the PARI SINUS combination of the PARI SINUS compressor and a jet nebuliser.

Another preferred aerosol generator includes an electronic vibrating-membrane type nebuliser such as the PARI eFlow. Unlike jet nebulisers, vibrating-membrane type nebulisers produce no air flow. For carrying out the present invention, an additional air flow from a compressor or another gas source is applied to the nebuliser in order to transport the aerosol from the nebuliser to the nose. This additional air stream may be continuous or intermittent. The pressure fluctuations (i.e. the pressure vibration or pulsation) are either superimposed to this air stream directly or via the outlet nostril, as described in co-pending European patent application no. 06025704.

Another preferred feature of the aerosol generator is that it is capable of emitting a pulsating aerosol at a rate of at least about 0.1 mL of dispersed liquid phase per minute. More preferably, the emission rate is at least about 0.125 mg/min, or at least about 0.15 mg/min. To achieve a particularly short duration of administration of the aerosol, other embodiments of the invention exhibit an aerosol generator capable of emitting at least about 0.175 g of dispersed liquid phase per minute, and in a further embodiment, the output rate is about 0.2 g/min or even higher.

As mentioned above, the aerosol deposition in the paranasal sinuses is also affected by individual anatomical features of the patient receiving the aerosol, such as the diameter of the respective ostia and the volume of the sinuses. In those many cases in which it is not feasible to adapt the aerosol parameters to a particular patient, the aerosol must be suitable for a wide variety of patient anatomies. It has been surprisingly found by the inventors that the anatomical differences are best taken into account by providing an aerosol whose geometrical particle size distribution is substantially wider than that which is usually deemed desirable for inhalation therapy. For example, an aerosol with a geometric standard deviation of the mass median diameter of less than about 2, which may be desirable for drug delivery targeted to the lungs, does not appear to be particularly useful for sinus delivery: such aerosol may be suitable for a subgroup of the patients, but rather unsuitable for others.

According to the findings of the inventors, better results may be obtained with aerosol generators based on jet nebulisers if the geometric standard deviation of the MMD of the aerosol is larger than about 2, such as about 2.3 or more. In other preferred embodiments, the geometric standard deviation is at least about 2.4, and at least about 2.5, and at least about 2.6, respectively. Other preferred geometric standard deviations range of about 2.4 to 2.7, and from about 2.5 to about 2.7, respectively.

By adapting the size distribution, it may be possible to achieve certain desired sinunasal distribution patters. For example, a relatively high mass median diameter of about 4 to 6 µm and a geometric standard deviation of 2.5 to 2.6 typically result in a rather high aerosol deposition in the nasal cavities, such as 20 to 50 % of the administered aerosol, and in a moderate paranasal sinus deposition, such as about 5 to 10 % of the aerosolised liquid phase. In contrast, an aerosol with a MMD of about 3 µm and a geometric standard deviation of only 2.4 to 2.5 typically leads to a much higher ratio of sinus delivery to nasal cavity delivery, such as between about 1 : 1 and 3:1, with about 3 to 6 % of the inhaled aerosol being deposited in the nasal cavities and about 5 to 15 % being deposited in the paranasal sinuses.

The invention is practised with any aerosolisable liquid comprising an active compound which is suitable for inhalation. In addition, the formulation should be designed and processed to be pharmaceutically acceptable. Most preferably, the liquid composition should be sterile when withdrawn from its packaging container. The inactive ingredients of the liquid composition should be pharmaceutically acceptable.

The liquid composition may of course comprise further excipients, such as one or more solvents, co-solvents, acids, bases, buffering agents, osmotic agents, stabilizers, antioxidants, taste-masking agents, flavours, sweetening agents, ionic surfactants, thickeners, colouring agents, fillers, and bulking agents.

Solvents and co-solvents, other than water, should be avoided if possible if the composition is intended for inhalation. If the incorporation of a solvent cannot be avoided, the excipient should be selected carefully and in consideration of its physiological acceptability. For example, if the composition is designated for the treatment of a life-threatening disease, the use of some limited amount of ethanol, glycerol, propylene glycol or polyethylene glycol as a non-aqueous solvent may be acceptable. According to the presently more preferred embodiments, however, the composition is substantially free of these solvents, and in particular of glycerol, propylene glycol or polyethylene glycol.

In order to provide a well tolerated aerosol, the preparation should be adjusted to a euhydric pH value. The term "euhydric" already implies that there may again be a divergence between pharmaceutical and physiological requirements so that a compromise has to be found which, for example, guarantees that the preparation is, from an economical point of view, just sufficiently stable during storage but, on the other hand, largely well tolerated. Preferably, the pH value lies in the slightly acidic to neutral region, i.e., between pH values of about 3.5 to 8.5. It is to be noted that deviations towards a weakly acidic environment can be tolerated better than shifts of the pH value into the alkaline region. A pH value in the range of about 4.5 to about 7.5 is particularly preferred.

For adjusting and, optionally, buffering pH value, physiologically acceptable acids, bases, salts, and combinations of these may be used. Suitable excipients for lowering the pH value or as acidic components of a buffer system are strong mineral acids, in particular, sulphuric acid and hydrochloric acid. Moreover, inorganic and organic acids of medium strength as well as acidic salts may be used, for example, phosphoric acid, citric acid, tartaric acid, succinic acid, fumaric acid, methionine, acidic hydrogen phosphates with sodium or potassium, lactic acid, glucuronic acid etc. However, sulphuric acid and hydrochloric acid are most preferred. Suitable for raising the pH value or as basic component for buffer system are, in particular, mineral bases such as sodium hydroxide or other alkali and alkaline earth hydroxides and oxides such as, in particular, magnesium hydroxide and calcium hydroxide, ammonium hydroxide and basic ammonium salts such as ammonium acetate, as well as basic amino acids such as lysine, carbonates such as sodium or magnesium carbonate, sodium hydrogen carbonate, citrates such as sodium citrate etc.

In one of the preferred embodiments, the liquid composition contains a buffer system consisting of two components, and one of the preferred buffer systems contains citric acid and sodium citrate. Nevertheless, other buffering systems may also be suitable.

Not primarily for physiological, but for pharmaceutical reasons, the incorporation of one or more excipients to achieve chemical stabilisation may be required. This depends mainly on the kind of the active agent contained therein. The most common degradation reactions of chemically defined active agents in aqueous preparations comprise, in particular, hydrolysis reactions, which may be limited, primarily, by optimal pH adjustment, as well as oxidation reactions. Examples for active agents which may be subject to oxidative attack are those agents that have olefinic, aldehyde, primary or secondary hydroxyl, ether, thioether, endiol, keto or amino groups. Therefore, in the case of such oxidation-sensitive active agents, the addition of an antioxidant, optionally in combination with a synergist, may be advisable or necessary.

Antioxidants are natural or synthetic substances which prevent or interrupt the oxidation of the active agents. These are primarily adjuvants which are oxidisable themselves or act as reducing agents, such as, for example, tocopherol acetate, reduced glutathione, catalase, peroxide dismutase. Synergistic substances are, for example, those which do not directly act as reactance in oxidation processes, but which counteract in oxidation by an indirect mechanism such as the complexation of metal ions which act catalytically in the oxidation, which is the case, for example, for EDTA derivatives (EDTA: ethylenediamine tetraacetic acid). Further suitable antioxidants are ascorbic acid, sodium ascorbate and other salts and esters of ascorbic acid (for example, ascorbylpalmitate), fumaric acid and its salts, malic acid and its salts, butyl hydroxy anisole, propyl gallate, as well as sulphites such as sodium metabisulfite. Apart from EDTA and its salts, citric acid and citrates, malic acid and its salts and maltol (3-hydroxy-2-methyl-4H-pyran-4-one) may also act as chelating agents.

In one of the embodiments, the composition contains at least one antioxidant. In a further embodiment, it contains both an antioxidant and a chelating agent. The combination of a vitamin E derivative, in particular, vitamin E acetate, with an EDTA derivative, in particular, EDTA disodium salt, is particularly preferred. In the case of certain active agents, this combination has proven to be particularly advantageous for obtaining high chemical stability and durability of the composition. In particular, in combination with the active agent budesonide, this combination of excipients is preferred.

In order to be well-tolerated, an aerosol should, as far as possible, have a physiologic tonicity or osmolality. Thus, it may be desirable to incorporate an osmotically active excipient to control the osmolality of the aerosol. The content of this excipient (or excipients, if a combination of substances is used) should be selected to yield an osmolality of the aerosol which does not deviate too much from that of physiological fluids, i.e., from about 290 mOsmol/kg. However, in individual cases, a compromise has again to be found between the physical-chemical or pharmaceutical needs on one hand and the physiological requirements on the other hand. Furthermore, it is believed that sinunasal aerosol delivery is not as problematic in terms of osmolality as, for example, deep lung delivery of aerosols. In general, an osmolality in the range of up to 800 mOsmol/kg may be acceptable. In particular, an osmolality in the range of about 200 up to about 550 mOsmol/kg is preferred. In further embodiments, the osmolality is even closer to the physiological value, i.e. from about 220 to about 400 mOsmol/kg.

If the active agent and the surfactants contained in the composition give an osmolality below the required or desired value it can be adjusted to the desired value by the addition of one or more suitable osmotically active excipients. Such compounds are, in particular, innocuous mineral salts which react largely neutrally (unless such adjuvants are, at the same time to adjust or buffer the pH value), such as sodium, calcium or magnesium chloride, sulfate or phosphate. One of the particularly preferred members of these is sodium chloride. Further preferred excipients for this purpose are magnesium and calcium sulfate and chloride.

As an alternative to the neutral mineral salts, physiologically safe organic compounds may be used as isotonising agent. Particularly suitable are water soluble substances with a relatively low molecular weight, for example, with a molecular weight of less than 300 or, better still, less than 200 and with a correspondingly high osmotic activity. Examples for such excipients are sugars and sugar alcohols, in particular, trehalose, mannitol, sorbitol and isomaltol.

Among the optional excipients are preservatives, which may be considered less desirable for aerosols which are for inhalation. Therefore, in one of the embodiments, the composition is substantially free of preservatives. However, if the composition, or a medicament comprising the composition, is to be packaged in multiple unit dose containers, it may be necessary that a preservative is used in order to maintain sterility.

In one of the preferred embodiments, the liquid from which the aerosol of the invention is obtained is provided in aqueous liquid form. Alternatively, it may be provided in form of a dry solid material which is adapted for preparing an aqueous liquid which can be administered as an aerosol. If the chemical and physical stability of the active agent and the composition permit, it is preferred that the composition is provided in liquid form. If an acceptable shelf life cannot be achieved, the composition must be formulated as a dry solid, such as a powder or lyophilisate for reconstitution.

As used herein, aqueous liquids are liquid compositions in which the liquid carrier or solvent consists predominantly of water, or at least 50 wt.-% of which represent water. The liquid state means that the preparation is either a liquid single-phase system or a multi-phase system but having a continuous liquid phase. Thus, the aqueous liquid according to the invention may represent an aqueous solution, a colloidal solution, a suspensions or an emulsion.

Even though the liquid carrier is predominantly water, it may, in individual cases, contain one or more liquids which are at least partially miscible with water, such as ethanol, glycerol, propylene glycol or polyethylene glycol. However, it is preferred that the composition is substantially free of non-aqueous liquids.

Even though the aerosolization of emulsions and suspensions is possible, the aqueous liquid preferably represents a solution, or a colloidal solution or dispersion, according to some of the embodiments of the invention. Colloidal solutions, or dispersions, are defined herein as monophasic systems, unlike e.g. suspensions which contain a dispersed solid phase. The rationale behind this is that the colloidal material dispersed within a colloidal solution or dispersion (as used herein, these are interchangeable) does not have the measurable physical properties usually associated with a solid material; furthermore, it does not provide a true solid-liquid interphase.

Colloidal carrier systems, such as micelles, mixed micelles, colloidal complexes, and liposomes, have been used in drug delivery as carriers for poorly water-soluble active compounds, or for the targeted delivery of certain drug substances.

In a colloidal system, not all components are molecularly dispersed; at least one of them is colloidally dispersed. Usually, colloidal structures are understood as being in a size range below about 1 µm, as commonly understood, or between 1 and about 500 nm as defined in other sources (H. Stricker, Physikalische Pharmazie, 3rd Edition, page 440). Therefore, colloidal structures are practically not visible with a light microscope and do not result in market turbidity of the solution, but rather in opalescence. However, the size limits given above are not rigid since they will depend to some extent on the properties under consideration. This nomenclature can be applied to coarser systems, especially when a gradual transition of properties is considered.

According to one of the embodiments of the invention, the liquid composition which is converted into an aerosol comprises a colloidal carrier system with an average size of up to about 1 µm (as measured by photon correlation spectroscopy). In further embodiments, the average diameter is from about 10 nm to about 400 nm. In a further embodiment, it is from about 10 nm to about 250 nm.

The colloidal structures should preferably have a relatively narrow size distribution. For example, if the composition contains liposomes and if it is intended to include a step of sterile filtration in the manufacture of the composition, the average diameter of the liposomes should preferably be below about 200 nm but also rather narrowly distributed in order to allow the sterile filtration procedure without problems such as drug loss or changes in the composition due to the retention of a substantial fraction of larger liposomes. A suitable parameter describing the distribution of the diameter of the colloidal structures is the polydispersity index. It is preferred that the polydispersity index is below about 0.5. More preferably, the polydispersity index is below about 0.3. In a further embodiment, it is below 0.2, such as about 0.1 to about 0.1, or even below 0.1.

A relatively low polydispersity index, reflecting a narrow size distribution, can be achieved by methods generally known to technically qualified persons. For example, liposomal solution may be sonicated, homogenized (optionally with the use of high pressure), or extruded through membranes under moderate pressure. Dialysis or centrifugation can be used as methods to isolate more narrow fractions of colloidal structures.

The respective compositions are not only characterized by the presence of colloidal structures, but also by the low content or even absence of larger particles. In particular, larger particles capable of sedimentation, or particles of solid material should preferably be absent.

Colloidal structures of various types are known to exist in different types of colloidal liquids. In isotropic colloidal solutions, the properties of the solution are the same regardless of the direction of measurement. In other words, in the isotropic state, all directions are indistinguishable from each other. For example, a micellar solution may be isotropic. In anisotropic colloidal solutions, there is orientation and/or alignment of molecules which causes the physical properties of the solution to vary for different directions of measurement. Such anisotropic solutions are referred to as liquid crystals, or liquid-crystalline phases, or mesophases.

Although the inventors do not wish to be bound by a particular theory, it is believed that, depending on the selected type, content and ratio of excipients, in particular of the non-ionic surfactant component and the phospholipid component, various colloidal structures may be generated within the aqueous system of the composition.

In some cases, for example, micelles or mixed micelles may be formed by the surfactants, in which poorly soluble active agents can be solubilised. In general, micelles are understood as substantially spherical structures formed by the spontaneous and dynamic association of amphiphilic molecules, such as surfactants. Mixed micelles are micelles composed of different types of amphiphilic molecules. Both micelles and mixed micelles should not be understood as solid particles, as their structure, properties and behaviour are much different from solids. The amphiphilic molecules which form the micelles usually associate temporarily. In a micellar solution, there is a dynamic exchange of molecules between the micelle-forming amphiphile and monomolecularly dispersed amphiphiles which are also present in the solution.

The position of the drug molecules which are solubilised in such micelles or mixed micelles depends on the structure of these molecules as well as the surfactants used. For example, it is to be assumed that particularly non-polar molecules are localized mainly inside the colloidal structures, whereas polar substances are more likely to be found on the surface.

If the liquid composition represents as a micellar or mixed micellar solution, it is preferred that the average size of the micelles is less than about 200 nm (as measured by photon correlation spectroscopy), such as from about 10 nm to about 100 nm. Particularly preferred are micelles with average diameters of about 10 to about 50 nm.

Methods for the preparations and characterization of liposomes and liposome preparations are known as such to the skilled person. Often, multilamellar vesicles will form spontaneously when amphiphilic lipids are hydrated, whereas the formation of small unilamellar vesicles usually requires a process involving substantial energy input, such as ultrasonication or high pressure homogenization. Further methods for preparing and characterizing liposomes have been, for example, described by S. Vemuri et al. [Preparation and characterization of liposomes as therapeutic delivery systems: a review. Pharm Acta Helv. 1995, 70(2):95-111].

Of the known liposomes, those are preferred according to the invention which have a predominantly colloidal size, i.e., whose average particle size lies below about 1 µm, and better still at maximally about 500 nm. Highly preferred is a diameter of up to about 200 nm. Such average particle size will usually allow sterile filtration through a filter with a pore size of 0.22 µm, which is a significant advantage in case the composition is not stable enough to withstand heat sterilization.

To obtain the aerosol of the invention which is suitable for nasal, paranasal sinus, or sinunasal delivery, the surface tension of the composition of the invention should preferably be adjusted to the range of about 25 to 80 mN/m, and preferably to the range of about 30 to 75 mN/m. In this context, it is to be taken into consideration that, in the lowest part of this range, a particularly good spreadability of the preparation on the mucous membranes may be expected, but that the quality of the aerosol and the efficiency of the nebulization could be adversely affected.

On the other hand, if a surfactant is incorporated in order to colloidally solubilise a poorly soluble active agent, it can hardly be avoided that the surface tension is reduced fairly markedly below that of water or physiological buffer solution. Thus, a compromise may have to be found in each case depending on the active compound and the intended application.

Moreover, it has surprisingly been found that, contrary to the findings of prior art such as WO 01/02024, a surface tension which is lower than that of water or aqueous buffer solutions is not necessary for sinunasal aerosol deposition. In fact, the present inventors have found that liquid compositions having a relatively high surface tension can be effectively delivered to the mucosal surfaces of the nasal cavity and of the paranasal sinuses if the teachings of the present invention are observed, and if the aerosol is designed to exhibit the features claimed herein. A low surface tension may be unavoidable if a surface-active drug substance or excipient is incorporated in the liquid compositions which is aerosolised, but if no surfactant is required and if the drug substance itself does not lead to a marked decrease of surface tension, it is preferred according to the present invention that the surface tension is selected in the region of about 65 to about 80 mN/m, such as in the range of approx. 70 mN/m.

The dynamic viscosity also has an influence on the particle size distribution of the aerosol formed by nebulisation and on the efficiency of nebulisation. It should preferably be adjusted to a range of about 0.8 to about 3 mPas. According to another embodiment, the dynamic viscosity is in the range of about 1.0 to about 2.5 mPas, or in the range from about 1.2 to about 2.0 mPas.

The active compound comprised in the aerosol of the invention is typically a drug substance which is useful for the prevention, management, or treatment of an affection, condition, symptom, or disease of, or related to, the mucosa of the nasal cavity and/or of the paranasal sinuses, for example acute and chronic sinusitis, such as allergic sinusitis, seasonal sinusitis, bacterial sinusitis, fungal sinusitis, viral sinusitis, frontal sinusitis, maxillary sinusitis, sphenoid sinusitis, ethmoid sinusitis, vacuum sinusitis; acute and chronic rhinitis, such as allergic rhinitis, seasonal rhinitis, bacterial rhinitis, fungal rhinitis, viral rhinitis, atrophic rhinitis, vasomotor rhinitis; any combination of rhinitis and sinusitis (i.e. rhinosinusitis); nasal polyps, nasal furuncles, epistaxis, wounds of the nasal or sinunasal mucosa, such as after injury or surgery; and dry nose syndrome.

Among the active compounds which may be useful for serving one of these purposes are, for example, substances selected from the groups of anti-inflammatory compounds, glucocorticoids, anti-infective agents, antibiotics, antifungals, antivirals, mucolytics, antiseptics, vasoconstrictors, wound healing agents, local anaesthetics, peptides, and proteins.

Examples of potentially useful anti-inflammatory compounds are glucocorticoids and non-steroidal anti-inflammatory agents such as betamethasone, beclomethasone, budesonide, ciclesonide, dexamethasone, desoxymethasone, fluoconolone acetonide, flucinonide, flunisolide, fluticasone, icomethasone, rofleponide, triamcinolone acetonide, fluocortin butyl, hydrocortisone, hydroxycortisone-17-butyrate, prednicarbate, 6-methylprednisolone aceponate, mometasone furoate, elastane, prostaglandin, leukotriene, bradykinin antagonists, non-steroidal anti-inflammatory drugs (NSAIDs), including any pharmaceutically acceptable salts, esters, isomers, stereoisomers, diastereomers, epimers, solvates or other hydrates, prodrugs, derivatives, or any other chemical or physical forms of active compounds comprising the respective active moieties.

Examples of anti-infective agents, whose class or therapeutic category is herein understood as comprising compounds which are effective against bacterial, fungal, and viral infections, i.e. encompassing the classes of antimicrobials, antibiotics, antifungals, antiseptics, and antivirals, are
- penicillins, including benzylpenicillins (penicillin-G-sodium, clemizone penicillin, benzathine penicillin G), phenoxypenicillins (penicillin V, propicillin), aminobenzylpenicillins (ampicillin, amoxycillin, bacampicillin), acylaminopenicillins (azlocillin, mezlocillin, piperacillin, apalcillin), carboxypenicillins (carbenicillin, ticarcillin, temocillin), isoxazolyl penicillins (oxacillin, cloxacillin, dicloxacillin, flucloxacillin), and amiidine penicillins (mecillinam);
- cephalosporins, including cefazolins (cefazolin, cefazedone); cefuroximes (cerufoxim, cefamdole, cefotiam), cefoxitins (cefoxitin, cefotetan, latamoxef, flomoxef), cefotaximes (cefotaxime, ceftriaxone, ceftizoxime, cefmenoxime), ceftazidimes (ceftazidime, cefpirome, cefepime), cefalexins (cefalexin, cefaclor, cefadroxil, cefradine, loracarbef, cefprozil), and cefiximes (cefixime, cefpodoxim proxetile, cefuroxime axetil, cefetamet pivoxil, cefotiam hexetil), loracarbef, cefepim, clavulanic acid / amoxicillin, Ceftobiprole;
- synergists, including beta-lactamase inhibitors, such as clavulanic acid, sulbactam, and tazobactam;
- carbapenems, including imipenem, cilastin, meropenem, doripenem, tebipenem, ertapenem, ritipenam, and biapenem;
- monobactams, including aztreonam;
- aminoglycosides, such as apramycin, gentamicin, amikacin, isepamicin, arbekacin, tobramycin, netilmicin, spectinomycin, streptomycin, capreomycin, neomycin, paromoycin, and kanamycin;
- macrolides, including erythromycin, clarythromycin, roxithromycin, azithromycin, dithromycin, josamycin, spiramycin and telithromycin;
- gyrase inhibitors or fluroquinolones, including ciprofloxacin, gatifloxacin, norfloxacin, ofloxacin, levofloxacin, perfloxacin, lomefloxacin, fleroxacin, garenoxacin, clinafloxacin, sitafloxacin, prulifloxacin, olamufloxacin, caderofloxacin, gemifloxacin, balofloxacin, trovafloxacin, and moxifloxacin;
- tetracyclins, including tetracyclin, oxytetracyclin, rolitetracyclin, minocyclin, doxycycline, tigecycline and aminocycline;
- glycopeptides, inlcuding vancomycin, teicoplanin, ristocetin, avoparcin, oritavancin, ramoplanin, and peptide 4;
- polypeptides, including plectasin, dalbavancin, daptomycin, oritavancin, ramoplanin, dalbavancin, telavancin, bacitracin, tyrothricin, neomycin, kanamycin, mupirocin, paromomycin, polymyxin B and colistin;
- sulfonamides, including sulfadiazine, sulfamethoxazole, sulfalene, co-trimoxazole, co-trimetrol, co-trimoxazine, and co-tetraxazine;
- azoles, including clotrimazole, oxiconazole, miconazole, ketoconazole, itraconazole, fluconazole, metronidazole, tinidazole, bifonazol, ravuconazol, posaconazol, voriconazole, and ornidazole and other antifungals including flucytosin, griseofluvin, tonoftal, naftifin, terbinafin, amorolfin, ciclopiroxolamin, echinocandins, such as micafungin, caspofungin, anidulafungin;
- nitrofurans, including nitrofurantoin and nitrofuranzone;
- polyenes, including amphotericin B, natamycin, nystatin, flucocytosine;
- other antibiotics, including tithromycin, lincomycin, clindamycin, oxazolindiones (linzezolids), ranbezolid, streptogramine A+B, pristinamycin aA+B, Virginiamycin A+B, dalfopristin /qiunupristin (Synercid), chloramphenicol, ethambutol, pyrazinamid, terizidon, dapson, prothionamid, fosfomycin, fucidinic acid, rifampicin, isoniazid, cycloserine, terizidone, ansamycin, lysostaphin, iclaprim, mirocin B17, clerocidin, filgrastim, and pentamidine;
- antivirals, including aciclovir, ganciclovir, birivudin, valaciclovir, zidovudine, didanosin, thiacytidin, stavudin, lamivudin, zalcitabin, ribavirin, nevirapirin, delaviridin, trifluridin, ritonavir, saquinavir, indinavir, foscamet, amantadin, podophyllotoxin, vidarabine, tromantadine, and proteinase inhibitors;
- antiseptics, including acridine derivatives, iodine-povidone, benzoates, rivanol, chlorhexidine, quarternary ammonium compounds, cetrimides, biphenylol, clorofene, and octenidine;
- plant extracts or ingredients, such as plant extracts from chamomile, hamamelis, echinacea, calendula, papain, pelargonium, essential oils, myrtol, pinen, limonen, cineole, thymol, mentol, camphor, tannin, alpha-hederin, bisabolol, lycopodin, vitapherole;
- wound healing compounds including dexpantenol, allantoin, vitamins, hyaluronic acid, alpha-antitrypsin, anorganic and organic zinc salts/compounds, salts of bismuth;
- interferones (alpha, beta, gamma), tumor necrosis factors, cytokines, interleukines;
- immunmodulators including methotrexat, azathioprine, cyclosporine, tacrolimus, sirolimus, rapamycin, mofetil;
- cytostatics and metastasis inhibitors;
- alkylants, such as nimustine, melphanlane, carmustine, lomustine, cyclophosphosphamide, ifosfamide, trofosfamide, chlorambucil, busulfane, treosulfane, prednimustine, thiotepa;
- antimetabolites, e.g. cytarabine, fluorouracil, methotrexate, mercaptopurine, tioguanine;
- alkaloids, such as vinblastine, vincristine, vindesine;
- antibiotics, such as alcarubicine, bleomycine, dactinomycine, daunorubicine, doxorubicine, epirubicine, idarubicine, mitomycine, plicamycine;
- complexes of secondary group elements (e.g. Ti, Zr, V, Nb, Ta, Mo, W, Pt) such as carboplatinum, cis-platinum and metallocene compounds such as titanocendichloride;
- amsacrine, dacarbazine, estramustine, etoposide, beraprost, hydroxycarbamide, mitoxanthrone, procarbazine, temiposide;
- paclitaxel, iressa, zactima, poly-ADP-ribose-polymerase (PRAP) enzyme inhibitors, banoxantrone, gemcitabine, pemetrexed, bevacizumab, ranibizumab.

Examples of potentially useful mucolytics are DNase, P2Y2-agonists (denufosol), heparinoids, guaifenesin, acetylcysteine, carbocysteine, ambroxol, bromhexine, tyloxapol, lecithins, myrtol, and recombinant surfactant proteins.

Examples of potentially useful vasoconstrictors which may be useful to reduce the swelling of the mucosa are phenylephrine, naphazoline, tramazoline, tetryzoline, oxymetazoline, fenoxazoline, xylometazoline, epinephrine, isoprenaline, hexoprenaline, and ephedrine.

Examples of potentially useful local anaesthetic agents include benzocaine, tetracaine, procaine, lidocaine and bupivacaine.

Examples of potentially useful antiallergic agents include the afore-mentioned glucocorticoids, cromolyn sodium, nedocromil, cetrizin, loratidin, montelukast, roflumilast, ziluton, omalizumab, Heparinoids and other antihistamins, Azelastine, Cetirizin, Desloratadin, Ebastin, Fexofenadin, Levocetirizin, Loratadin.

Examples of potentially useful peptides and proteins include antibodies against toxins produced by microorganisms, antimicrobial peptides such as cecropins, defensins, thionins, and cathelicidins.

For any of these and other explicitly mentioned examples of drug substances which are potentially useful for carrying out the invention, the compound names given herein should be understood as also referring to any pharmaceutically acceptable salts, solvates or other hydrates, prodrugs, isomers, or any other chemical or physical forms of the respective compounds comprising the respective active moieties.

Examples of potentially useful mucolytics are acetylcysteine, carbocysteine, ambroxol, bromhexine, tyloxapol, and recombinant surfactant proteins.

Examples of potentially useful vasoconstrictors which may be useful to reduce the swelling of the mucosa are phenylephrine, naphazoline, tramazoline, tetryzoline, oxymetazoline, fenoxazoline, xylometazoline, epinephrine, isoprenaline, hexoprenaline, and ephedrine.

Examples of potentially useful local anaesthetic agents include benzocaine, tetracaine, procaine, lidocaine and bupivacaine.

Examples of potentially useful antiallergic agents include the afore-mentioned glucocorticoids, cromolyn sodium, and nedocromil.

Examples of potentially useful peptides and proteins include antibodies against toxins produced by microorganisms, antimicrobial peptides such as cecropins, defensins, thionins, and cathelicidins.

However, the practice of the invention is not limited to these compounds. Any therapeutic or prophylactic substance or mixture or combination of substances having a potentially beneficial effect on the mucosa of the nasal cavity and/or of the paranasal sinuses should be understood as within the scope of the invention.

In one of the embodiments, the active compound is selected from drug substances which have poor water solubility, such as a solubility in water of less than about 1 wt.-% at 20 °C. In another embodiment, the solubility of the active compound, or of one of the active compounds present in the liquid phase of the aerosol, is less than about 1 mg/mL.

Poorly water-soluble active agents are generally not very easy to administer as aerosolised liquids, one of the reasons being that the volume of liquid which can be administered as an aerosol is limited. However, it has been found by the inventors that, surprisingly, such compound may be delivered in aerosolised form even to the sinunasal mucosa if the teachings and preferences disclosed herein are observed.

Poor water solubility is particularly problematic if the drug substance also requires relatively large unit doses. As mentioned above, a unit dose is understood as the quantity of an active compound which is suitable and effective when given at one event of administration. Optionally, a unit dose, or single dose, is administered repeatedly, according to a certain regimen, such as once daily, twice daily, or three times daily, for an extended period of time, such as several days, weeks, or even longer.

If, for example, a drug substance is not dissolvable in 5 mL of water or of an aqueous carrier, oral administration or parenteral infusion may still be feasible because large volumes of water (or gastrointestinal fluid) are available to dissolve the compound and render it absorbable. In contrast, it is more difficult to formulate such compounds for aerosol delivery, because the volume of liquid carrier cannot be increased ad libitum. In the case of sinus or sinunasal aerosol delivery, it has been found by the inventors that unit doses having a volume of more than 5 mL lead to large losses of drug, as the nasal and sinus mucosa can only be wetted by a small amount of liquid.

While it may be difficult to formulate such compounds as aerosolisable liquid compositions for nasal and/or sinus delivery, the problems can be overcome by using drug nanoparticles, by combining the active compound with a colloidal carrier system, or by solubilising it with a solubility-enhancing agent or excipient.

In one of the embodiments, the a unit dose of the active compound requires more than about 5 mL of water to be dissolved at 20 °C, and is incorporated within the liquid from which the aerosol of the invention is obtained in the form of nanoparticles, incorporated within or associated with a colloidal carrier, or in solubilised form, wherein the solubilised form is achieved through the incorporation of a solubility-enhancing agent. In other embodiments, the aqueous solubility of the active ingredient relative to its unit dose is still lower, such as requiring at least about 10 mL of water to be dissolved at 20 °C, or more than about 50 mL, or even more than about 100 mL.

As used herein, nanoparticles are particles of a semisolid or solid material having a diameter in the range of up to about 1 µm. It should be noted that the solid state may be difficult to observe for such small particles, and no solid-liquid-interphase may be detectable in liquid systems comprising such nanoparticles. Nevertheless, the material from which the nanoparticles are predominantly composed is a semisolid or solid material under normal conditions. Nanoparticles may have various shapes and structures: nanospheres, nanorods, and nanocapsules are only a few examples of different types of nanoparticles. In one of the preferred embodiments, the nanoparticles have a mass median diameter of less than about 800 nm, and in a further embodiment, the mass median diameter is less than about 600 nm or even less than about 500 nm. In further embodiments, the mass median diameter is in the range from about 150 to about 450 nm.

If nanoparticles are present in the liquid phase of the aerosol, they are preferably stabilised by at least one excipient which is optionally selected from the group of surfactants, polyelectrolytes, and thickeners or gelling agents. The function of the stabiliser or stabilisers is to prevent the agglomeration, or at least the irreversible agglomeration of the nanoparticles, which have a particularly high surface energy.

Preferably, the nanoparticles are predominantly composed of the active compound, but covered with at least a layer of stabiliser molecules. Further optional features of drug nanoparticles which can be incorporated within liquid compositions which are suitable for aerosolisation are disclosed, for example, in WO 96/25918, whose teachings are incorporated herein by reference.

Colloidal carriers, or colloidal drug carriers, are structures in the colloidal size range, i.e. typically having an average diameter of less than about 1 µm, which may be primarily composed of excipient molecules. In such colloidal constructs, a drug substance may be incorporated, or an active ingredient may simply be associated with the colloidal carrier. Non-limiting examples of such colloidal carriers include liposomes, lipid complexes, micelles, mixed micelles, lipid nanoparticles, nanoparticles, nanocapsules, niosomes, and polymer conjugates. Further aspects of such colloidal systems have been described herein-above.

Optionally, the liquid phase of the aerosol comprises a poorly water-soluble active agent and a solubility-enhancing excipient, such as a surfactant, a base, an acid, or a complexing agent, such as a cyclodextrin. As used herein, a solubility-enhancing agent is an excipient or a combination of excipients whose presence in an aqueous liquid composition, such as the liquid phase of the aerosol of the invention, results in a substantially enhanced molecular or colloidal solubility of the incorporated active ingredient. In particular, a solubility-enhancing agent or excipient effects an increase in the solubility of the active compound of at least 20 %. In further embodiments, the increase in solubility, whether molecular or colloidal, is at least about 50 %, at least about 100 %, and at least about 150 %, respectively. Preferably, the solubility-enhancing agent is selected in quality and quantity to achieve that a unit dose of the active compound, which may not be dissolvable in 5 mL of water in the absence of the solubility-enhancing agent at 20 °C, is now dissolved or colloidally solubilised in a liquid volume of not more than about 5 mL, and preferably in a liquid phase whose volume is not more than about 4 mL. According to another embodiment, the active agent is dissolved or colloidally solubilised in a liquid whose volume is not more than about 3 mL, such as from about 2 mL to about 3 mL.

Optionally, the solubility-enhancing agent adjusts the pH of an aqueous liquid composition to a value at which the active compound is better soluble. In this case, the solubility-enhancing agent is selected from the group of pharmaceutically acceptable acids and bases. As used herein, the terms acid and base include acidic and basic salts or, more generally defined, compounds whose saturated aqueous solution exhibits a pH which is substantially different from 7, such as below about 6 for acids, and above about 8 for bases.

Examples of pharmaceutically acceptable acids and bases include inorganic excipients such as ammonium salts, sodium hydroxide, magnesium hydroxide, calcium hydroxide, sulphuric acid, hydrochloric acid, phosphoric acid; and organic compounds such as lysine, methionine, arginine, citric acid, and fumaric acid.

Alternatively, the solubility-enhancing agent is a pharmaceutically acceptable surfactant. Surfactants are amphiphilic, surface- or interface-active materials. Such compounds have at least one relatively hydrophilic and at least one relatively hydrophobic, or lipophilic, molecular region. They accumulate at phase interfaces and reduce surface tension. Surfactants are often used, inter alia, in order to stabilize multi-phase systems. Non-ionic surfactants are surfactants which have no real ionic charge in aqueous media at substantially neutral pH (for example, between pH 4 and 10), but, at most, partial charges. A surfactant may also be referred to as a detergent or tenside, or, to denote its function in particular compositions, as an emulsifier or wetting agent.

Suitable non-ionic surfactants include, in particular, those which are to be considered safe for oral or nasal inhalation or oromucosal administration. Examples of non-ionic surfactants which appear to have a particularly good physiological compatibility are tyloxapol, polysorbates such as polysorbate 80, vitamin E-TPGS, and macrogol hydroxystearates such as macrogol-15-hydroxystearate.

Optionally, more than one surfactant may be present in the liquid phase which is aerosolised, such as polysorbate 80 in combination with vitamin E-TPGS. It has been observed that the effect of surfactants on the properties of the composition, in particular the solubilising effect on poorly soluble active agents, can be additive. This means that by incorporating two surfactants instead of only one can achieve a desired effect on the formulation at a lower concentration of each of the surfactants, which may be useful to avoid the occurrence of adverse effects caused by a higher content of only one particular surfactant.

Phospholipids are an example of ionic surfactants. They may be defined as amphiphilic lipids which contain phosphorus. Also known as phosphatides, they play an important role in nature, in particular, as double layer-forming constituents of biological membranes. Phospholipids which are chemically derived from phosphatidic acid occur widely and are also commonly used for pharmaceutical purposes. This acid is a usually (doubly) acylated glycerol-3-phosphate in which the fatty acid residues may be of different length. The derivatives of phosphatidic acid include, for example, the phosphocholines or phosphatidylcholines, in which the phosphate group is additionally esterified with choline, furthermore phosphatidyl ethanolamines, phosphatidyl inositols etc. Lecithins are natural mixtures of various phospholipids which usually have a high proportion of phosphatidyl cholines. Depending on the source of a particular lecithin and its method of extraction and/or enrichment, these mixture may also comprise significant amounts of sterols, fatty acids, triglycerides and other substances.

Suitable phospholipids are also those which are suitable for administration by inhalation on account of their physiological properties. These comprise, in particular, phospholipid mixtures which are extracted in the form of lecithin from natural sources such as soy beans or chickens egg yoke, preferably in hydrogenated form and/or freed from lysolecithins, as well as purified, enriched or partially synthetically prepared phospholipids, preferably with saturated fatty acid esters. Particularly preferred are purified, enriched or partially synthetically prepared medium-to long-chain zwitterionic phospholipids which are mainly free from unsaturation in the acyl chains and free from lysolecithins and peroxides. Of the phospholipid mixtures, lecithin is particularly preferred. Examples for enriched or pure compounds are dimyristoyl phosphatidyl choline (DMPC), distearoyl phosphatidyl choline (DSPC) and dipalmitoyl phosphatidyl choline (DPPC). Of these, DMPC is currently more preferred. Alternatively, phospholipids with oleyl residues and phosphatidyl glycerol without choline residue are suitable for some embodiments and applications of the invention.

Other ionic surfactants which may serve as solubility-enhancing agents are, for example, sodium lauryl sulfate, sodium cetylstearyl sulfate, sodium (or calcium or potassium) docusate, medium and long chain fatty acids, etc.

Other solubility-enhancing agents may be selected from various chemical groups, such as co-solvents, chaotropic salts, urea, and complexing agents. Particularly preferred among these are complexing agents, especially compounds from the class of pharmaceutically acceptable cyclodextrins.

Cyclodextrins (CDs) are cyclic oligosaccharides composed of (α-1,4)-linked α-D-glucopyranose units. They comprise a relatively hydrophobic central cavity and a hydrophilic external region: Because the monomeric units cannot rotate freely at the α-1,4-linkages, the shape of the molecules is more conical than cylindrical, with the primary hydroxyl groups located at the smaller part and the secondary hydroxyl groups at the larger part of the conus.

The most common cyclodextrins are α-, β-, and γ-cyclodextrins with 6, 7, and 8 glucopyranose units, respectively. The diameter of the cavities are approximately 4.7 to 5.3 A for α-cyclodextrins, 6.0 to 6.5 for β-cyclodextrins, and 7.5 to 8.3 for γ-cyclodextrins. The non-derivatised cyclodextrins exhibit aqueous solubilities of about 145 mg/mL (α-cyclodextrin), 18.5 mg/mL (β-cyclodextrin), and 232 mg/mL (y-cyclodextrin) at 25 °C.

Cyclodextrins are known for their capability of forming inclusion complexes with smaller molecules. If the host molecules themselves are poorly water-soluble, they may become solubilised in the form of such cyclodextrin inclusion complexes. Several pharmaceutical agents have been successfully formulated into marketed drug products which incorporate cyclodextrins as solubility-enhancing agents.

Examples of potentially useful cyclodextrins include the non-derivatised cyclodextrins, but also derivatives whose hydroxyl groups are alkylated or hydroxyalkylated, esterified, or etherified, such as 2-hydroxypropyl-β-cyclodextrin, 2-hydroxypropyl-γ-cyclodextrin, sulfobutyl-β-cyclodextrin, sulfobutyl-γ-cyclodextrin, maltosyl-β-cyclodextrin, and methyl-β-cyclodextrin. Particularly preferred at present are 2-hydroxypropyl-β-cyclodextrin, and sulfobutyl-β-cyclodextrin, α-cyclodextrin, β-cyclodextrin, and γ-cyclodextrin.

According to another embodiment, the poorly soluble active compound is enhanced in solubility by the presence of at least two solubility-enhancing excipients, such as a cyclodextrin and a surfactant, or a non-ionic and an ionic surfactant, a surfactant and an acid or base, or a cyclodextrin and an acid or base. Of course, more than two solubility-enhancing agents may also be combined and incorporated in the liquid formulation from which the aerosol is obtained, if such number of excipients is needed and appears pharmaceutically acceptable.

Depending on the physical and chemical stability of the liquid composition, a commercially useful shelf life may not be achievable. For example, if the active compound is hydrolytically labile, it is possible that an aqueous liquid formulation with a shelf life of at least two years cannot be successfully formulated. As a further example, physically complex carrier systems, such as surfactant-stabilised nanoparticulate systems or colloidal drug carriers like liposomes may not be physically stable over a sufficient period of time to ensure the required shelf life.

In any of these cases, it may be useful to develop a solid-state formulation which can be reconstituted into a liquid composition prior to administration. The solid formulation comprises at least the active compound or, if the liquid phase of the aerosol is to comprise more than one active compound, the solid formulation comprises at least one of the active compounds.

Depending on the design and composition of the aerosol's dispersed liquid phase, the solid composition for reconstitution may comprise further ingredients which may or may not be selected from any of the solid excipients disclosed herein-above. Among the preferred excipients are osmotic agents, such as inorganic salts; excipients for adjusting or buffering the pH, such as organic or inorganic salts, acids, and bases; bulking agents and lyophilisation aids, such as sucrose, lactose, mannitol, sorbitol, xylitol, and other sugar alcohols; stabilisers and antioxidants, such as vitamin E or vitamin E derivatives, ascorbic acid, sulphites, hydrogen sulphites, gallic acid esters, butyl hydroxyanisole, and butyl hydroxytoluene; ionic and nonionic surfactants, including phospholipids, such as those surfactants disclosed above; complexing agents, such as cyclodextrins, in particular those cyclodextrins that are disclosed above; furthermore taste-masking agents, disintegrants, colouring agents, sweeteners, and flavours.

The solid composition for reconstitution may be part of a pharmaceutical kit. Such kit preferably comprises the solid composition in sterile form. As used herein, As used herein, the term "sterility" is to be defined according to the usual pharmaceutical meaning. It is understood as the absence of germs which are capable of reproduction. Sterility is determined with suitable tests which are defined in the relevant pharmacopoeias. According to current scientific standards, a sterility assurance level of 10⁻⁶ is generally regarded as acceptable for sterile preparations, i.e., one unit in a million might be contaminated.

In practice, however, contamination rates may be higher. For example, it is generally assumed that the contamination rate for aseptically manufactured preparations might amount to about 10⁻³. Since, on one hand, the extent of sterility tests for quality control of lots according to the pharmacopeias is limited and, on the other hand, contaminations may be caused as artefacts while carrying out the test itself, it is difficult to demand sterility in an absolute sense or to test a particular product for it. Therefore, the sterility of the composition should be understood herein such that the composition meets the requirements with respect to sterility of the relevant pharmacopeia. The same applies to the liquid formulations which are ready to use.

The solid composition may be prepared by providing a liquid composition which is similar to the liquid composition to be aerosolised, and subsequently drying it, such as by lyophilisation. Similar means that the liquid composition from which the solid composition is prepared by drying may not comprise all solid ingredients of the ready-to-use liquid composition, for example in the case that the liquid carrier for reconstitution is designed to comprise one or more of the excipients. Also, it is not necessary that the concentrations of the ingredients are identical for these two liquid compositions.

Alternatively, the solid composition for reconstitution may be prepared by providing the active ingredient and, optionally, at least one excipient, in powder form and subsequently mixing these to form a powder mixture.

The solid composition is preferably packaged in closed containers each of which holds the amount of the formulation which contains a unit dose of the active compound. Alternatively, but presently less preferred because of the risk of microbial contamination, a container holds a plurality of unit doses. Additionally, the kit may comprise a liquid carrier for reconstituting the solid composition and for preparing a liquid composition for aerosolisation, wherein the aerosol is used for delivering the active compound(s) to the mucosa of the nose or of one or more paranasal sinuses.

According to one embodiment, such liquid carrier may consist of water only. In other embodiments, the carrier also comprises one or more physiologically inactive ingredients, such as one or more excipients selected from buffers and pH-adjusting agents, salts, surfactants etc. The liquid carrier may be provided in separate packaging containers holding the amount of liquid which is needed for reconstituting an amount of solid formulation containing one unit dose. If the solid formulation is packaged in containers holding more than a unit dose, the liquid carrier may also packaged in larger containers. In this case, either the liquid carrier or the solid composition should also comprise a preservative, or a combination of preservatives, to prevent microbial growth after reconstitution.

The containers comprising the liquid carrier and the solid composition for reconstitution may comprise means for connecting them to each other in order to facilitate the combination of their respective contents and enable easy and convenient reconstitution. In one of the embodiments, the liquid carrier and the solid composition are filled in two separate chambers of a dual chamber device adapted to separately store the two components and to mix them on demand without having to withdraw them from the containers. In this way, reconstitution may be more convenient and can be conducted without microbial contamination.

Alternatively, the kit may comprise one or more means or devices which are adapted to withdraw the liquid carrier or to mix the two components conveniently and effectively. Preferably, the kit also contains printed instructions on how to reconstitute the formulation and on how the reconstituted liquid composition is to be aerosolised and administered in order to achieve the delivery of the active compound to the mucosa of the nasal cavity and/or of the paranasal sinuses.

As mentioned above, the liquid composition is for aerosolisation and for the prevention, management, or treatment of an affection, condition, symptom, or disease of, or related to, the mucosa of the nasal cavity and/or of the paranasal sinuses, for example acute and chronic sinusitis, such as allergic sinusitis, seasonal sinusitis, bacterial sinusitis, fungal sinusitis, viral sinusitis, frontal sinusitis, maxillary sinusitis, sphenoid sinusitis, ethmoid sinusitis, vacuum sinusitis; acute and chronic rhinitis, such as allergic rhinitis, seasonal rhinitis, bacterial rhinitis, fungal rhinitis, viral rhinitis, atrophic rhinitis, vasomotor rhinitis; any combination of rhinitis and sinusitis (i.e. rhinosinusitis); nasal polyps, nasal furuncles, epistaxis, wounds of the nasal or sinunasal mucosa, such as after injury or surgery; and dry nose syndrome. A particularly preferred use of the aerosol, and thus of the liquid composition from which the aerosol is obtained, is for the treatment of acute and chronic forms of sinusitis.

Preferably, the use involves repeated administration, optionally according to a regular administration regimen over a period of at least three days, with at least one administration per day. According to another embodiment, the administration frequency is about twice or thrice a day. Optionally, the treatment involves a more frequent administration, such as 4 times a day or more often. If once daily administration is selected, it is preferred that the actual time interval between two consecutive administrations is in the range from about 18 to 30 hours, such as every evening, every noon, or every morning, in order to avoid long periods without medication.

A unit dose of the active ingredient(s) is preferably formulated to a volume of 5 mL of liquid phase or less. As has been pointed out above, larger volumes bring about the risk of substantial drug losses due to the limited capacity of the sinunasal mucosa to hold the administered liquid. Moreover, large volumes typically require long administration times, which are inconvenient to the patient.

In order to provide a convenient and effective method of administering the aerosol of the invention, it is preferred that an aerosol generator is selected for aerosolising the liquid composition which is not only capable of emitting a pulsating aerosol, and which preferably maintains an amplitude of pulsation of at least about 5 mbar, but which is also capably of emitting at least about 0.1 mL of dispersed liquid phase per minute. According to another embodiment, the aerosol generator is adapted to emit at least about 0.15 mL of dispersed phase per minute, or at least about 0.175 mL per minute, such as about 2 mL/min or more. While this is not a particularly restrictive selection criterion for non-pulsating aerosol generators, it has been found by the inventors that not very many of the commercially available jet nebulisers are capable of such emission rates when combined with an air compressor which delivers a pulsating stream of air, such as the PARI SINUS compressor. Preferred nebulisers include, for example, the optionally adapted members of the PARI LC Sprint and the PARI LL families.

According to another embodiment, the volume of the liquid which comprises a unit dose and the output rate of the aerosol generator are selected in such a way that the administration time of a unit dose is not more than about 30 minutes, and more preferably not more than about 20 minutes. According to other embodiments, the administration time is not more than about 15 minutes, not more than about 12 minutes, and not more than about 10 minutes, respectively. For example, if the liquid composition is formulated to contain a unit dose within a particularly low volume, such as about 2 mL, and the output rate of the aerosol generator is particularly high, such as at least about 0.2 g/min, these short - and even shorter - administration times can be achieved.

For the avoidance of misunderstandings, it is pointed out that in common practice, the nominal unit dose is not completely aerosolised by aerosol generators such as jet nebulisers driven by air compressors, due to the typically presence of a dead volume in the nebuliser. The residual liquid in the device is often in the range from about 0.5 to about 1 mL. Therefore, the actually emitted volume of aerosolised liquid is less than the volume of liquid filled into the device, and - again according to common practice - the actually emitted dose of the active ingredient is less than the nominal dose filled into the device. Therefore, the time values given herein for the preferred duration of administering a unit dose should be understood as referring to the duration of aerosolising that fraction of the unit dose formulation which is actually emitted, excluding the fraction of the liquid and of the drug substance which is lost in form of a residue in the device.

The invention is further illustrated by the following examples which should not be understood as limiting the scope of the invention as claimed in the patent claims.

### EXAMPLES

### Example 1 (comparative example)

A commercial aqueous suspension formulation of fluticasone-17-propionate (Flutide^{®} forte Fertiginhalat 2,0 mg/2 ml) was nebulised with an aerosol generator (SinuNEB^{™}) which has been suggested for the delivery to the paranasal sinuses. The sinunasal deposition of the aerosol was evaluated in an *in vitro* model.

*Sinunasal deposition model.* A cast model based on the anatomical shapes and dimensions of the nasal cavity and the nasal passage was built from plastic. In this model, the paranasal sinuses are simulated by 6 exchangeable glass bottles, 3 on either side, representing the frontal, maxillary, and sphenoid sinuses, respectively. Exchangeable, artificial ostiae of 10 mm length were used to connect the artificial sinus cavities to the nose model. Moreover, the model has two openings representing artificial nostrils and one opening for the simulation of the pharynx which connects the nasal cavity with the trachea. The deposition model is also equipped with a pressure sensor inside the nasal cavity in order to determine the amplitude of the aerosol pressure pulsation.

The configuration used for this experiment included an internal volume of 7.5 ml for each of the frontal sinuses, 23 ml for each of the maxillary sinuses, and of 13 ml for each of the sphenoid sinuses. The diameter of the ostiae was 0.5 mm for the frontal sinuses, 2 mm for the maxillary sinuses, and 1 mm for the sphenoid sinuses. The interior space of each of the glass bottles representing the sinuses was lined with a patch of filter material.

*Test formulation.* Flutide^{®} forte Fertiginhalat 2,0 mg/2 ml is a commercial aqueous suspension formulation of fluticasone-17-propionate, comprising polysorbate 20, sorbitan laurate, sodium dihydrogenphosphate dihydrate, disodium hydrogenphosphate, sodium chloride, and water for injection as inactive ingredients. According to the label, the concentration of the active ingredient is 1.0 mg/ml. The product is approved for administration by inhalation, using a jet nebuliser, for the treatment of mild to moderate asthma.

*Test procedure.* A SinuNEB^{™} aerosol generator, which generates a non-pulsating aerosol, was connected tightly with the sinunasal deposition model so that the aerosol emitted from the device would flow into the artificial nostrils of the model. The artificial pharynx was connected via an inspiratory filter unit with a PARI breath simulator.

A volume of 2.0 ml of Flutide^{®} forte aqueous suspension containing 2.070 µg of fluticasone-17-propionate (according to an assay performed with an equivalent volume of the same batch) was filled into the nebuliser of the SinuNEB^{™} aerosol generator. The nebuliser was weighed accurately. For 8 minutes, the aerosol generator and the breath simulator were operated simultaneously, the latter with a breathing pattern characterised by a tidal volume of 600 ml, 12 breaths per minute, and an inhalation to exhalation ratio of 3 : 2, representing a typical adult breathing pattern. Subsequently, the artificial sinuses together with the ostiae, the nasal cavity, and the inspiratory filter unit were carefully rinsed with defined volumes of solvent, which were analysed for fluticasone-17-propionate. Once more, the nebuliser was weighed.

In result, it was found that the residual fluticasone-17-propionate in the nebuliser after the nebulisation period was 1.740 µg, which was about 84 % of the charged amount of drug. A fraction of 89 µg - or about 4.3 % - was found in the inspiratory filter unit, representing the amount of drug which may be deposited in the lower respiratory system and/or exhaled. The combined paranasal sinuses held 0.44 µg of active ingredient, which is about 0.02 % of the charged dose. An amount of 9.5 µg or about 0.5 % was deposited in the remaining areas of the sinunasal model, i.e. in the nasal cavity.

### Example 2 (comparative example)

To exclude the possibility that an aqueous suspension such as Flutide^{®} forte - even though the product is approved for administration by inhalation using a jet nebuliser - is particularly unsuitable for aerosol delivery to the sinuses, the experiment of Example 1 was repeated with a different formulation of fluticasone-17-propionate. In this case, the test formulation was an experimental aqueous solution which contained 455 µg of the drug in the charged volume of 2.0 ml. Gamma-cyclodextrin (50 mg/ml) was incorporated to solubilise the poorly water-soluble active ingredient. Further inactive ingredients were sodium chloride (9 mg/ml) and hydrochloric acid (to pH 6). The dynamic viscosity of the solution was 1.14 mPa·s, the surface tension 71.6 mN/m.

In result, the relative amount of drug remaining in the nebuliser was lower (about 56 % of the charged dose) and the inhaled fraction was higher (about 16.2 %) than in the case of Flutide^{®} forte. However, the deposition in the sinuses (about 0.04 %) and in the nasal cavity (about 0.7 %) remained insignificant. The total output rate, which is the rate by which the aerosol is emitted from the apparatus, was 129.3 mg/min.

### Example 3

In another experiment, the same sinunasal deposition model and the same test formulation as in Example 1 were used, but a different aerosol generator. Thus, instead of a SinuNEB^{®} aerosol generator, a VibrENT^{®} apparatus was used, which is a jet nebuliser similar to the PARI LC Star^{®}, but adapted to deliver an aerosol into a nostril, in combination with a compressor (PARI SINUS) designed to deliver compressed air whose pressure pulsates at a frequency of 30 to 60 Hz. A frequency of 44 Hz was selected.

Another difference was that the nosepiece of the nebuliser was connected with one of the nostrils at a time, and that the other nostril was closed with a flow resistor. Aerosol, coming out of the exit nostril was captured on a filter. The artificial pharynx was closed, taking into account that the VibrENT^{®} aerosol generator is intended for nasal inhalation with the soft palatine being in the closed position, thus separating the sinunasal space from the lower respiratory tract. The total nebulisation time was the same as in Example 1, i.e. 8 minutes, or 4 minutes for each nostril.

The mass median diameter of the aerosol, which had been determined separately by laser diffraction, was 3.82 µm, with a geomteric standard deviation of 2.24.

In result, it was found that 1.760 µg, or 85 % of the charged dose of active ingredient, were left in the nebuliser; 99 µg (4.8 %) were found in the exhalation filter unit. Total sinus deposition was 38.7 µg (1.9 %), and drug deposition in the nasal cavity was 66.8 µg (3.2 %). Thus, the sinunasal deposition was found to be substantially higher than in Example 1, which is not an example following the teachings of the present invention. In particular, the deposition in the artificial sinuses was increased by a factor of 88. The total output rate was 172 mg/min.

### Example 4

The experiment of Example 3 was repeated, except that the test formulation was the same as in Example 2, i.e. an experimental aqueous solution of fluticasone-17-propionate with a drug content of 455.0 µg in a charged volume of 2.0 ml. The mass median diameter of the aerosol droplets was 3.37 µm. The geometric standard deviation was 2.31.

In result, the deposition of this aerosol in the sinuses was 29.0 µg (6.4 % of the charged dose), and nasal deposition was 28.4 µg (6.2 %). The residual drug in the nebuliser was 245.0 µg (53.8 %), and the exhaled fraction was 109 µg (24.0 %). The total output rate was 173 mg/min.

### Example 5

The experiment of Example 3 was repeated, except that a different aerosol generator was used, which was now a novel combination of a jet nebuliser unit similar to the PARI LC Sprint Junior, but adapted to deliver a pulsating aerosol into a nostril, and a PARI SINUS compressor. A frequency of 44 Hz was selected.

The device emitted an aerosol having a mass median diameter of 3.04 µm and a geometric standard deviation of 2.42. Sinus deposition was 69.9 µg, or 3.4 % of the charged dose of fluticasone-17-propionate, and deposition on the nasal cavity was 83.5 µg (4.0 %). The total output rate was 171 mg/min.

### Example 6

The same basic assembly of the aerosol generator and sinunasal deposition model was used as in Example 3.

The volumes of the artificial sinuses were 23 ml (maxillary), 7.5 ml (frontal) and 13 ml (sphenoid); the respective ostiae had the diameters of 2.0 mm, 0.5 mm, and 1.0 mm (same order). The pulsation frequency of the compressor was set at 44 Hz.

The test formulation was an aqueous ambroxol hydrochloride solution with a drug content of 7.6 mg/ml. The solution further comprised sodium chloride. It exhibited a pH of 5.92 at 22 °C, an osmolality of 0.299 mOsmol/kg, a dynamic viscosity of 1.04 mPa·s, and a surface tension of 70.76 mN/m. The fill volume was 3.0 ml. The emitted aerosol droplets had a mass median diameter between 3 and 4 µm. Nebulisation time was 5 minutes for each of the nostrils.

Of the charged dose of ambroxol hydrochloride, 1.62 mg (7.1 %) were deposited in the artificial paranasal sinuses, and 2.44 mg (10.7 %) were found in the nasal cavity. Thus, the total sinunasal deposition was 4.1 mg (17.8 %), with a ratio of 0.66 : 1 of sinus to nasal deposition. The exhaled fraction was 6.69 mg (29.3 %), and the residual drug in the nebuliser amounted to 10.84 mg (47.5 %).

### Example 7

The experiment of Example 6 was repeated with the following variations. First of all, a brand-new nebuliser unit of the same type was used. The fill volume was reduced to 2.5 ml, and the nebulisation time was reduced to 4 minutes for each nostril. The mass median diameter of the aerosol was 3.2 µm, and the total output rate about 200 mg/min. The aerosol vibrated with a mean pressure amplitude of about 40 mbar.

The deposited amounts of ambroxole hydrochloride were 1.2 mg (6.3 %) for the sinuses and 1.3 mg (6.8 %) for the nasal cavity. The filter unit contained 6.07 mg (31.9 %) and the residual drug in the nebuliser was 9.69 mg (51 %). Thus, the total sinunasal deposition was 2.5 mg (13.2 %), with a ratio of roughly 1 : 1 of sinus to nasal deposition.

### Example 8

In another set of experiments, two different PARI LC Sprint nebulisers were used, one of which was specifically modified to generate a coarser aerosol. Using the ambroxole hydrochloride solution of Example 7, the unmodified nebuliser emitted aerosol droplets with a mass median diameter of 2.7 µm with a geometric standard deviation (GSD) of 2.38, the modified device exhibited a diameter of 5.7 µm with a GSD of 2.63. The nebulisers were driven by a PARI Universal compressor adapted to deliver compressed air which may pulsate at various frequencies.

In a set of experiments, various sinus volumes (between 7.5 and 23 ml), ostia diameters (0.5 to 2.0 mm), and pulsation frequencies (30 to 60 Hz) were selected and tested for each of the two nebulisers. As the modified nebuliser showed a higher total output rate, it was filled with 4.0 ml of the test formulation instead of with 2.0 ml as in the case of the non-modified device. Nebulisation time was 5 minutes for each nostril with the regular nebuliser and 3 minutes per nostril for the adapted device. All aerosols vibrated with a pressure amplitude in the range from 21 to 30 mbar.

Summarising the results, it was found that, in average (for all model settings), the finer aerosol led to a sinus deposition of 10.3 % of the emitted aerosol (636 µg), whereas 7.2 % (672 µg) of the coarser aerosol was deposited in the sinuses. The deposition in the nasal cavity was 6.3 % (389 µg) for the fine aerosol and 45.9 % (4283 µg) for the coarse aerosol. Surprisingly, the absolute amounts of drug deposited in the sinus cavities were similar for the fine as well as for the coarse aerosol, the difference regarding the deposition relative to the emitted aerosol was small.

The experiments demonstrate how a larger mass median diameter of the aerosol droplets may be used to shift the deposition pattern towards a higher ratio of nasal to sinus deposition, such as from below 1 (1:1) to more than 6 (6 : 1). Therefore, relatively low aerosol droplet diameters, such as in the region of roughly 3 µm, appear particularly suitable for treating condisitions which primarily affect the paranasal sinuses, whereas relatively larger droplet, such as roughly in the region of 5 to 6 µm, may be particularly suitable for therapies in which a high exposition of the nasal mucosa to the drug is also desirable.

### Example 9

The aqueous solution of a therapeutic protein (DNAse, 1.0 mg/ml) was nebulised with the aerosol generator described in Example 5. The test solution exhibited an osmolality of 285 osmol/kg, a pH of 6.5, a surface tension of 72.1 mN/m, and a viscosity of 0.98 mPa·s.

The sinunasal cast model was configured as in example 6. A fill volume of 2.5 ml was charged. The device emitted an aerosol with a mass median diameter of 3.19 and a geometric standard deviation of 2.50. The nebulisation time was 4 minutes per nostril. The mean total output rate was 215 mg/min.

It was found that, in average, 7.5 % of the charged drug was deposited in the artificial sinus cavities. Another 11.6 % were found in the remaining parts of the sinunasal model, i.e. in the nasal cavity. The exhaled drug fraction was 36.5 %, and the residual drug in the nebuliser amounted to 40.5 %.

### Example 10

An aqueous solution of Levofloxacin (5 mg/ml) was nebulised with the aerosol generator described in Example 3 (VibrENT).

The sinunasal cast model was configured as follows (sinus position / ostium diameter / sinus volume): frontal right / 3.0 mm / 7.5 ml; frontal left /1.0 mm /7.5 ml; maxillary right / 3.0 mm /23 ml; maxillary left 1.0 mm / 23 ml; sphenoid right /3.0 mm /12 ml; sphenoid left 1.0mm / 12 ml. A fill volume of 3.0 ml was charged. The device emitted an aerosol with a mass median diameter of 3.4 µm and a geometric standard deviation of 2.4. The nebulisation time was 4 minutes per nostril. The mean total output rate was 170 mg/min.

It was found that, in average, 2.9 % of the charged drug was deposited in the artificial sinus cavities. Another 6.0 % were found in the remaining parts of the sinunasal model, i.e. in the nasal cavity. The exhaled drug fraction was 21.8%, and the residual drug in the nebuliser amounted to 69 %.

The deposited absolute amounts of the single sinus cavities were as follows: frontal right: 23.6 µg; frontal left: 71.4µg, maxillary right: 145.3 µg, maxillary left: 86.1 µg, sphenoid right: 53.1 µg and sphenoid left: 74.2 µg.

Interestingly, there was no simple relationship between the ostium diameter and the deposited amount of drug. Only in the maxillary cavities with a relatively large volume of 23 ml, the deposition was higher for the larger ostium diameter than for the small ostium diameter. In human anatomy, there appears to be a substantial variation of sinunasal geometries, including varying ostium diameters and sinus volumes. Based on this variety, it appears favourable to use aerosols having a relatively broad particle size distribution.

### Example 11

Aerosol characteristics of Mucosolvan, a commercially available ambroxol solution (7.5 mg/ml) with a surface tension of 35.3 mN/m, an osmolarity of 309 mOsmol/kg, a pH of 5.5 and a viscosity of 1.04 mPa s were compared to the ambroxol solution of example 6. The only significant physical difference between these solutions is their surface tension, which is relatively low in the case of Mucosolvan which contains the surface-active ingredient benzalkonium chloride as preservative, while the ambroxol test solution is preservative free and has a surface tension of 70 mN/m. The solutions were aerosolized via a LC STAR nebuliser, driven by a PARI Boy N compressor. Measurements were performed with a Malvern Mastersizer X laser diffraction instrument.

Mucosolvan aerosol had a mean MMD of 3.22 µm and a geometric standard deviation (GSD) of 2.04. Amroxol test solution had a MMD of 3.16 µm and a GSD of 2.14. Statistic data analysis (ANOVA) showed significant dependence of GSD on formulation (p = 0.0054). The results indicate that a higher surface tension appears to increase the GSD. As Aerosols with a relatively high GSD are preferred for sinus drug delivery, it is preferred that the composition comprises little or no surfactant, unless a surfactant is required due to the specific formulation needs of an active compound.

## Claims

1. Method for producing a pharmaceutical aerosol for the delivery of an active compound to the mucosa of the nose or of a paranasal sinus, said aerosol comprising a dispersed liquid phase and a continuous gas phase, said method comprising:
(a) providing an aerosol generator capable of emitting an aerosol whose pressure pulsates with a frequency in the range from about 10 to about 90 Hz, wherein the aerosol generator is adapted to maintain an amplitude of pressure pulsation of the emitted aerosol of at least about 5 mbar,
(b) providing a liquid composition comprising said active compound, wherein a unit dose of the active compound is comprised in a volume of less than about 5 mL of said liquid composition, and
(c) aerosolising said liquid composition.

2. The method of claim 1, wherein the aerosol generator is capable of emitting an aerosol whose pressure pulsates with a frequency in the range from about 25 to about 80 Hz, and preferably from about 35 to about 60 Hz.

3. The method of claim 1 or 2, wherein the aerosol generator is adapted to maintain an amplitude of pressure pulsation of the emitted aerosol of at least about 10 mbar.

4. The method of any of the preceding claims, wherein the aerosol generator is capable of emitting the aerosol at a rate of at least about 0.1 g/min, and preferably of at least about 0.15 g/min.

5. The method of any of the preceding claims, wherein a unit dose of the active compound is comprised in a volume of the liquid composition of less than about 4 mL, and preferably of less than about 2.5 mL.

6. The method of any of the preceding claims, wherein the aerosol generator is capable of emitting a quantity of aerosol comprising a unit dose of the active compound within less than about 10 minutes.

7. The method of any of the preceding claims, wherein the mass median diameter of the dispersed liquid phase is from about 2.0 to about 6.0 µm, as measured by laser diffraction.

8. The method of any of the preceding claims, wherein geometric standard deviation of the mass median diameter of the dispersed liquid phase is at least about 2.3.

9. The method of any of the preceding claims, wherein the active compound is a member of the group of: anti-inflammatory compounds, glucocorticoids, anti-allergies, antioxidants, vitamins, leucotriene antagonists, anti-infective agents, antibiotics, antifungals, antivirals, mucolytics, decongestants, antiseptics, cytostatics, immunomodulators, would healing agents, local anaesthetics, peptides, and proteins.

10. The method of any of the preceding claims, wherein the active compound has a water solubility of less than about 1 mg/mL at 20 °C, or wherein a unit dose of the active compound requires more than about 5 mL of water to be dissolved at 20 °C.

11. The method of claim 10, wherein the dispersed liquid phase comprises a solubility-enhancing agent.

12. The method of claim 11, wherein the solubility-enhancing agent is selected from the group of surfactants, acids, bases, complexing agents, in particular cyclodextrins or polymeric compounds in particular chitosan and hydroxypropylmethylcellulose.

13. The method of claim 10, wherein the active compound is in the form of nanoparticles.

14. The method of claim 10, wherein the dispersed liquid phase comprises a colloidal carrier system, preferably selected from the group of liposomes, lipid complexes, micelles, mixed micelles, lipid nanoparticles, nanoparticles, nanocapsules, niosomes, and polymer conjugates.

15. The method of any of the preceding claims, wherein the liquid composition contains an antibiotic in combination with an antifungal and/or antiviral compound.

16. The method of any of claims 1 to 14, wherein the liquid composition contains an anti-inflammatory drug in combination with an antibiotic, antifungal and/or antiviral compound.

17. The method of any of the preceding claims, wherein the liquid composition exhibits a dynamic viscosity in the range from about 0.8 to about 3 mPas.

18. The method of any of the preceding claims, wherein the liquid composition exhibits a surface tension in the range from about 25 to about 80 mN/m.

19. The method of any of the preceding claims, wherein the step of providing the liquid composition comprising the active compound comprises:
(a) providing a solid composition comprising said active compound,
(b) providing a liquid for reconstituting said solid composition, and
(c) reconstituting said solid composition with said liquid to obtain a liquid composition comprising the active compound.

20. The method of any of the preceding claims, wherein the aerosol generator includes a nebuliser selected from the group consisting of jet nebulisers and electronic vibrating membrane nebulisers.

21. A pharmaceutical aerosol prepared by the method of any of claims 1 to 20.

22. The use of the pharmaceutical aerosol of claim 21 for administration at a frequency of at least once, twice or three times weekly, or once daily, over a period of at least three days.
